# EUROPEAN PATENT SPECIFICATION

(11) **EP 2 578 670 B1**
(45) Date of publication and mention of the grant of the patent: **25.09.2019**
(21) Application number: 11789933.6
(22) Date of filing: 03.06.2011
(51) Int. Cl.: A23K 20/111

(54) **CASHEW NUT SHELL OIL HAVING IMPROVED STABILITY**
CASHEWNUSSSCHALENÖL MIT ERHÖHTER STABILITÄT
BAUME DE CAJOU AYANT UNE STABILITÉ AMÉLIORÉE

(30) Priority: 03.06.2010 JP 2010128201
(43) Date of publication of application: 10.04.2013
(73) Proprietor: Idemitsu Kosan Co., Ltd., Chiyoda-ku Tokyo 100-8321 (JP)
(72) Inventor: ITO, Shinji, Sodegaura-shi Chiba 299-0293 (JP); NAGASHIMA, Kyo, Sodegaura-shi Chiba 299-0293 (JP); MOCHIZUKI, Masami, Sodegaura-shi Chiba 299-0293 (JP); OOIWA, Seika, Sodegaura-shi Chiba 299-0293 (JP)
(74) Representative: Vossius & Partner Patentanwälte Rechtsanwälte mbB
(86) International application number: PCT/JP2011/062829
(87) International publication number: WO 2011/152532

(56) References cited:
- WO-A1-99/10293
- WO-A1-2006/003668
- WO-A1-2009/139468
- CA-A- 464 500
- CA-A- 464 500
- GB-A- 259 959
- GB-A- 481 960
- GB-A- 536 800
- GB-A- 664 169
- GB-A- 664 169
- JP-A- 10 182 549
- JP-A- 2006 111 839
- JP-A- 2006 111 839
- JP-A- 2010 043 045
- JP-A- 2010 059 070
- JP-A- 2010 088 363
- JP-A- 2010 088 363
- US-A- 2 067 919
- US-A- 2 611 715
- US-A- 2 611 715
- US-A1- 2008 226 760
- DATABASE WPI Week 200570 Thomson Scientific, London, GB; AN 2005-678563 & JP 2005 239992 A (YASUHARA CHEM KK) 8 September 2005 (2005-09-08)
- DATABASE BIOSIS [Online] BIOSCIENCES INFORMATION SERVICE, PHILADELPHIA, PA, US; 2003, AGOSTINI-COSTA T S ET AL: "[Determination of anacardic acids in cashew apples (Anacardium occidentale L.).]", Database accession no. PREV200300342250
- Mahesh Venkatachalam ET AL: "Chemical Composition of Selected Edible Nut Seeds", Journal of Agricultural and Food Chemistry, vol. 54, no. 13, 1 June 2006 (2006-06-01), pages 4705-4714, XP055380650, ISSN: 0021-8561, DOI: 10.1021/jf0606959

## Description

### Technical Field

The present invention relates to a method of producing a non-heated cashew nut shell liquid (CNSL) preparation, in which a decarboxylation of an anacardic acid in the non-heated cashew nut is inhibited by an acid, and
a method of inhibiting a decarboxylation reaction of an anacardic acid in a non-heated cashew nut shell liquid, comprising adding an acid to the non-heated cashew nut shell liquid.

### Background Art

A cashew nut shell liquid is an oily liquid contained in a shell of a fruit of a cashew nut tree (*Anacardium occidentale L*.). The cashew nut shell liquid mainly contains, as its components, an anacardic acid, cardanol, cardol, and methylcardol.

A method of preparing a cashew nut shell liquid includes a heating method and a solvent-extraction method, but in general, the cashew nut shell liquid is used after converting the anacardic acid into a cardanol by heat treatment at a production district of the cashew nut.

This is because the anacardic acid that is a component of the non-heated cashew nut shell liquid easily decarboxylates and may form a foam during transportation. Further, the non-heated cashew nut shell liquid solidifies at about 20°C and loses its fluidity, and hence it is difficult to transport the liquid. For that reason, the transportation of the non-heated cashew nut shell liquid is largely constrained, which prevents further industrial uses.

Patent Documents 1 to 3 describe examples of applications for industrial products, but all the cashew nut shell liquids used are heat-treated products.

Patent Documents 4 to 6 mention the applications of the non-heated cashew nut shell liquid and the anacardic acid as a component of the liquid for feed. Because of the above-mentioned restrictions of stability and transportation, it is difficult to realize the applications including solving physical and economical problems. Further, Patent Documents 4 to 6 describe the applications of the non-heated cashew nut shell liquid, but there are no findings which focus on improvement in its stability and handling and seek to improve the stability and handling.

Accordingly, it has been desired to develop a method of stabilizing the non-heated cashew nut shell liquid so that the liquid can easily be transported.

Patent Document 7 describes a method of stabilizing an anacardic acid by adding an alkaline to a cashew nut shell liquid containing the anacardic acid and mixing the resultant. However, the document 7 does not describe the stabilization of the anacardic acid by adding an acid to the cashew nut shell liquid. Further, the document 7 does not describe the solidification of the non-heated cashew nut shell liquid at about 20°C can be prevented by blending a carrier in the non-heated cashew nut shell liquid supplemented with an acid.

Patent Document 8 relates to a process of separating the acid constituent from cashew nut shell liquid or hydrogenated cashew nut shell liquid.

Patent Document 9 discloses a particular process for the production of a treated cashew nut shell liquid which comprises intimately mixing raw cashew nut shell liquid with a dilute aqueous solution of an acidic agent.

Patent Document 10 relates to the preparation of products utilizing cashew nut shell liquid, and relates especially to the preparation of such products in combination with a modifying additive whereby thickening of the cashew nut shell liquid is promoted.

Patent Document 11 discloses an antimicrobial composition comprising in weight compared to the total mixture mass (a) about 2 to 100% of one or more hydroxylated fatty acids having 10 to 20 atoms of carbon in its chain, or one or more triglycerides containing one or more hydroxylated fatty acids having 10 to 20 atoms of carbon in its chain; (b) about 0 to 95% of one or more cashew compounds or compounds from Anacardiaceae family's species, or compounds selected from the group consisting of: cardol, cardanol, anacardic acid, and anacardic derivatives; and optionally (c) about 0 to 95% of one or more organic acids having 2 to 20 atoms of carbon in its chains.

Patent Document 12 discloses a process for forming a bonded product in which particles are mixed with cashew nut shell liquid and an acid to form a uniform mixture, and the mixture is then compacted under pressure in a mould.

Patent Document 13 relates to the polymerization of cashew nut shell liquid by reacting it with from about 1 % to about 6 % of its volume of concentrated sulphuric acid.

Patent Document 14 relates to reaction products of the oil from the shell and about the kernel of the cashew nut and to methods of producing them.

Patent Document 15 relates to the manufacture of frictional elements such as brakes, clutches, belting, pulley facings, tyre treads and other bodies used for the regulation and transmission of mechanical movement by means of frictional engagement.

Patent Document 16 discloses a process for the preparation of polyurethane polyol from cardanol and rigid foams therefrom comprising oxidising technical grade cashew nut shell liquid containing cardanol as the main component (> 95%) with a peracid generated in situ from hydroperoxide and an organic acid in presence of a catalyst, in order to oxidise the unsaturation in the side chain of cardanol, the ratio of cardanol to hydroperoxide employed being 1:2, the ratio of cardanol to organic acid is 1:1, the ratio of cardanol to organic acid to hydroperoxide being 1:1:2, the reaction temperature being in the range of 0°C and 80°C, maintaining the mixture at the reaction temperature to produce a hydroxy-formoxy ester, subjecting the hydroxy-fonnoxy ester to hydrolysis with sodium acetate to produce the polyol, and separating the polyol from the unreacted reactants.

Patent Document 17 relates to the polymerization of cashew nut shell liquid by means of chemicals to obtain products of several kinds which range in consistency from the liquid state to a rubber-like state.

Patent Document 18 relates to a polylactic acid-based resin composition containing a polylactic acid-based resin and (B) an esterified cashew nut shell liquid and/or a hydrogenated product of the esterified cashew nut shell liquid.

Agostini-Costa et al. reported in Non-patent Document 1 on the determination of anacardic acids in cashew apples.

Mahesh Venkatachalam et al. reported in Non-patent Document 2 on the chemical composition of selected edible nut seeds.

### Prior Art Documents

### Patent Documents

Patent Document 1: JP 2008-144171 A
Patent Document 2: JP 2006-111839 A
Patent Document 3: JP 2003-252893 A
Patent Document 4: JP 2003-238400 A
Patent Document 5: JP 2001-151675 A
Patent Document 6: JP 8-231410 A
Patent Document 7: JP 2010-88363 A
Patent Document 8: CA 464,500
Patent Document 9: GB 664,169
Patent Document 10: US 2,611,715
Patent Document 11: US 2008/226760
Patent Document 12: WO 99/10293
Patent Document 13: GB 481,960
Patent Document 14: GB 259,959
Patent Document 15: GB 536,800
Patent Document 16: WO 2006/003668
Patent Document 17: US 2,067,919
Patent Document 18: JP 2005/239992

### Non-patent Documents

Non-patent Document 1: Agostini-Costa et al., Revista Brasileira de Plantas Medicinais April 2003.
Non-patent Document 2: Mahesh Venkatachalam et al., Journal of Agricultural and Food Chemistry 2006, 54(13), pages 4705 to 4714

### Summary of the Invention

An object of the present invention is to enable easy transportation of a non-heated cashew nut shell liquid, in particular, an anacardic acid as a component thereof without decarboxylation. Another object of the present invention is to enable easy transportation of a non-heated cashew nut shell liquid that solidifies at about 20°C even at a low temperature.

The inventors of the present invention have conducted intensive studies to solve the above-mentioned problems, and as a result, the inventors have found that the addition of an acid to the non-heated cashew nut shell liquid can inhibit the decarboxylation of an anacardic acid.

Further, the inventors of the present invention have found that the blending of a carrier such as a grain powder or silica with the non-heated cashew nut shell liquid having the decarboxylation inhibited through the addition of the acid enables the transportation of the non-heated cashew nut shell liquid without solidification even at a low temperature of about 20°C or less.

Thus, the inventors of the present invention have completed the present invention, which is as defined in the claims.

A summary of the present disclosure is as follows.
(1) A composition comprising one or more acids and a non-heated cashew nut shell liquid, wherein decarboxylation of an anacardic acid in the non-heated cashew nut is inhibited by the one or more acids.
(2) The composition according to (1), wherein the acid has a pKa of 4.5 or less.
(3) The composition according to (1) or (2), wherein an amount of the acid is 0.5 wt% or more with respect to the non-heated cashew nut shell liquid.
(4) A non-heated cashew nut shell liquid preparation, comprising the composition according to any one of (1) to (3) and one or more carriers.
(5) The non-heated cashew nut shell liquid preparation according to (4), wherein the carrier is silica.
(6) A feed comprising a non-heated cashew nut shell liquid having an inhibited decarboxylation of an anacardic acid, which is produced by adding the composition according to any one of (1) to (3) and/or the non-heated cashew nut shell liquid preparation according to (4) or (5).
(7) A method of inhibiting a decarboxylation reaction of an anacardic acid in a non-heated cashew nut shell liquid, comprising adding one or more acids to the non-heated cashew nut shell liquid.
(8) A method of producing a non-heated cashew nut shell liquid preparation according to (4) or (5), comprising mixing one or more acid components with one or more carriers and further mixing the carrier mixed with the acid component with the non-heated cashew nut shell liquid.
(9) A method of inhibiting a decarboxylation reaction of an anacardic acid, comprising adding one or more acids to the anacardic acid.

The addition of an acid to a non-heated cashew nut shell liquid can inhibit the decarboxylation of an anacardic acid that is a component of the non-heated cashew nut shell liquid. Further, the blending of the carrier with the non-heated cashew nut shell liquid supplemented with the acid can prevent the solidification at about 20°C. Accordingly, the non-heated cashew nut shell liquid can be easily used for industrial applications.

### Brief Description of the Drawings

FIG. 1 shows inhibition of decarboxylation reactions of non-heated cashew nut shell liquids in the cases of using 1 wt% various acids. The bar graphs show, in a descending order, cardanols 15:1, 15:2, and 15:3, and anacardic acids 15:1, 15:2, and 15:3.
FIG. 2 shows the inhibition of the decarboxylation reactions of the non-heated cashew nut shell liquids in the cases of using 10 wt% various acids. The bar graphs show, in a descending order, cardanols 15:1, 15:2, and 15:3, and anacardic acids 15:1, 15:2, and 15:3.
FIG. 3 shows the inhibition of the decarboxylation reactions of the non-heated cashew nut shell liquids in the cases of using various concentrations of sulfuric acids. The bar graphs in FIG 3A show, in a descending order, cardanol 15:1 and anacardic acid 15:1. The bar graphs in FIG 3B show, in a descending order, cardanol 15:3 and anacardic acid 15:3.
FIG. 4 shows the inhibition of the decarboxylation reactions in the non-heated cashew nut shell liquid preparations in the case of using a 10 wt% phosphoric acid (acid was preliminarily added to non-heated cashew nut shell liquid) . The bar graphs show, in a descending order, cardanol 15:3 and anacardic acid 15:3.
FIG. 5 shows the inhibition of the decarboxylation reactions in the non-heated cashew nut shell liquid preparations in the case of using the 10 wt% phosphoric acid (acid was added to carriers in advance) . The upper bar graphs show, in a descending order, cardanol 15:1 and anacardic acid 15:1, and the lower bar graphs show, in a descending order, cardanol 15:3 and anacardic acid 15:3.
FIG. 6 shows the inhibition of the decarboxylation reactions in feeds in the case of using the 10 wt% phosphoric acid. The bar graphs show, in a descending order, cardanol 15:3 and anacardic acid 15:3.

### Mode for carrying out the Invention

The method of producing
a non-heated cashew nut shell liquid preparation is characterized in that a decarboxylation of an anacardic acid in the non-heated cashew nut is inhibited by one or more specific acids.

The cashew nut shell liquid to be used in the present invention is an oily liquid contained in the shell of the seed of a cashew nut tree (*Anacardium occidentale L*.). The cashew nut shell liquid contains, as components thereof, anacardic acid, cardanol, cardol, and methylcardol.

Non-heated cashew nut shell liquid (hereinafter, referred to as a cashew nut shell liquid) extracted by compressing the shell of a cashew nut contains 55 to 80 mass% anacardic acid, 5 to 20 mass% cardanol, and 5 to 30 mass% cardol as described in J. Agric. Food Chem. 2001, 49, 2548-2551.

It should be noted that there are three kinds of anacardic acids: an anacardic acid having three double bonds at 8-, 11-, and 14-positions (hereinafter, referred to as anacardic acid 15:3); an anacardic acid having two double bonds at 8- and 11-positions (hereinafter, referred to as anacardic acid 15:2) ; and an anacardic acid having one double bond at 8-position (hereinafter, referred to as anacardic acid 15:1).

The present invention further includes a method of inhibiting a decarboxylation reaction of an anacardic acid, which is characterized by adding one or more particular acids in a specific amount to the anacardic acid.

The cashew nut shell liquid to be used in the present invention can be obtained as a vegetable oil extracted by compressing the shell of a cashew nut. Further, the cashew nut shell liquid can also be obtained by extracting, e.g., solvent-extracting a cashew nut shell. In addition, the cashew nut shell liquid can be obtained according to a method described in JP 08-231410 A, e.g., by a solvent extraction method.

The cashew nut shell liquid may also be a liquid obtained by pulverizing/crushing the shell of a cashew nut.

Further, a commercially available cashew nut shell liquid product may also be used.

The acid to be used in the present invention has a pKa of preferably 4.5 or less, more preferably 4.0 or less.

More specifically the one or more acids is/are selected from a formic acid, a malic acid, a citric acid, a succinic acid, a tartaric acid,
a nitric acid,
a malonic acid, a glutaric acid, an adipic acid, a fumaric acid, a maleic acid, a phthalic acid, an isophthalic acid, a phytic acid, a terephthalic acid, a lactic acid, a sulfamic acid, a hydroxyacetic acid, a phosphonic acid, and a gluconic acid. In the present invention, the pKa value refers to a value calculated at 25°C when water is used as a solvent.

The amount of the acid to be used is 0.5 wt% or more, preferably 1 to 20'wt% with respect to the cashew nut shell liquid (CNSL). It should be noted that, in the case where the pKa of the acid is higher than 2, it is necessary to increase the acid to be added. For example, the amount may be adjusted to about 5 to 10 wt%.

The composition can be produced by adding one or more acids to the non-heated cashew nut.

The non-heated cashew nut shell liquid preparation obtained by the method of the present invention is a preparation containing the composition and one or more carriers.

The composition may be mixed with the one or more carriers to produce the non-heated cashew nut shell liquid preparation.

Examples of an inorganic carrier include, but are not limited thereto, a silicic acid and a salt thereof (such as silica), vermiculite, diatomaceous earth, talc, kaolin, and bentonite.

In the cases where a silicic acid and a salt thereof are used as the inorganic carriers, the carriers preferably each have a specific surface area of 500 m³/g or less so as not to cause significant oxidation reactions. In the case of using silica as the inorganic carrier, the blending ratio (weight ratio) is preferably silica/non-heated cashew nut shell liquid=1/3.0 to 1/0.1. In the case of using another inorganic carrier, the same specific surface area and blending ratio as described above may be employed. It should be noted that the specific surface area of silica can be measured by a BET method.

It should be noted that grain powders or the below-mentioned feed component such as corn grain, corn powder, milo, soybean cake, oat, wheat flour short, wheat coarse flour, alfalfa, clover, defatted rice bran, white fish meal, fish meal, yeast, molasses, meat pieces, or bone meal may be used as an organic carrier.

The method of producing the non-heated cashew nut shell liquid preparation
containing a non-heated cashew nut shell liquid, one or more carriers, and one or more acid components comprises:
preliminarily mixing the acid component with the carrier; and further mixing the carrier mixed with the acid component with the non-heated cashew nut shell liquid. The effect of the acid component in the carrier can inhibit the decarboxylation of the non-heated cashew nut shell liquid in a state in which the acid is mixed with the carrier.

The non-heated cashew nut shell liquid preparation
may include one or more antioxidants in addition to a non-heated cashew nut shell liquid, a carrier, and an acid component. Examples thereof include ethoxyquin, t-butylhydroxytoluene, t-butylhydroxyanisole, t-butylhydroquinone, an ascorbic acid and esters thereof, Vitamin E, a gallic acid and esters thereof, an erythorbic acid, a chlorogenic acid, sulfite, thiosulfate, phosphite, hypophosphite, and phosphate.

The non-heated cashew nut shell liquid preparation
may include one or more antibiotics in addition to a non-heated cashew nut shell liquid, a carrier, and an acid component. Examples thereof include zinc bacitracin, avilamycin, alkyl trimethyl anmonium calcium tetracycline, efrotomycin, enramaycin, chlortetracycline, sedecamycin, Semduramicin, narasin, nosiheptide, virginiamycin, bicozamycin, flavophospholipol, polynactin, monensin, salinomycin, lasalocid, lysoceline, lonomycin, avoparcin, colistin sulfate, tylosin phosphate, amprolium-ethopabate, sulfaquinoxaline, morantel citrate, decoquinate, nicarbazin, halofuginone polystyrene-sulfonate, kitasamycin, tiopeptone, Destomycin A, Hygromycin B, and salts thereof.

The non-heated cashew nut shell liquid preparation
may be formulated into a powder formulation by containing a carrier such as grain powders or silica. That is, the non-heated cashew nut shell liquid preparation
can be produced by mixing the non-heated cashew nut shell liquid, the one or more carriers, the one or more acid components, and if necessary, one or more optional components and formulating the mixture into a powder formulation. Such powder formulation
can be used as a feed without mixing the formulation with another optional component.

The non-heated cashew nut shell liquid preparation
can be formulated into not only a powder formulation but also a granular formulation such as a pellet. In this case, not only the inorganic carrier and the acid component but also one or more hardened oils may be added to the non-heated cashew nut shell liquid. As the hardened oil, an oil obtained by hardening a palm oil, a soybean oil, a rapeseed oil, or the like is used. The melting point of the hardened oil is preferably 45 to 65°C. It should be noted that a general extruding granulator can be used for producing the preparation as a pellet.

The non-heated cashew nut shell liquid preparation
may be coated. For example, after granulation, the preparation can be coated with one or more coating agents selected from zein, shellac, hydroxypropylmethylcellulose (HPMC), pullulan, hemilose, glucose, lactose, trehalose, and starch. Alternatively, the preparation may be coated with a sheet containing the coating agents as components.

A feed containing the composition and/or the non-heated cashew nut shell liquid preparation can be provided.
The non-heated cashew nut shell liquid to be contained in the feed has the decarboxylation inhibited by the one or more acid components .

In the feed, the kind and blending ratio of the feed component to be blended with the composition and/or the cashew nut shell liquid preparation
are not particularly limited. The feed may be one conventionally given to animals. For example, the feed may be prepared using one or more feed components such as corn grain, corn powder, milo, soybean cake, oat, wheat flour short, wheat coarse flour, alfalfa, clover, defatted rice bran, white fish meal, fish meal, yeast, molasses, meat pieces, bone meal, calcium carbonate, dibasic calcium phosphate, yellow grease, vitamins, or minerals.

The feed can be produced by adding the cashew nut shell liquid preparation as it is to a feed component and mixing the resultant. On this occasion, when a powdery or solid cashew nut shell liquid preparation is used, the form of the cashew nut shell liquid preparation may be modified into a liquid form or a gel form for the purpose of facilitating the mixing process. In this case, one or more of the followings may be used as a liquid carrier: water; a vegetable oil such as soybean oil, rapeseed oil, or corn oil; liquid animal oil; and a water-soluble polymer compound such as polyvinyl alcohol, polyvinylpyrrolidone, or polyacrylic acid. Further, in order to keep the uniformity of the cashew nut shell liquid in the feed, the feed preferably contains one or more of alginic acid, sodium alginate, a xanthan gum, casein sodium, an arabic rubber, a guar gum, and a water-soluble polysaccharide such as tamarind seed polysaccharide.

The feed is suitable for breeding livestock such as cows, pigs, chickens, sheep, horses, and goats. The amount of feed ingested by an animal may be appropriately adjusted depending on the animal's species, body weight, age, sex, health condition, feed component, etc. In this case, the amount of cashew nut shell liquid contained in the feed is preferably 0.005 to 500 g per animal per day, more preferably 0.05 to 100 g per animal per day.

Any method usually used may be adopted as a method of feeding animals and a method of raising animals depending on the species of animals.

### Examples

### Example 1. Sample preparation

500 kg of cashew nut shells were purchased from Cashew Trading Co., Ltd., and the shells were compressed, thereby producing 158 kg of a cashew nut shell liquid (non-heated cashew nut shell liquid) .

Further, the following 2 grade silica were used as the silica to be used as the carrier.
Sipernat 22 (pH 6.5) (manufactured by Evonik Degussa Japan Co., Ltd.)
Carplex #1120 (pH 10.6) (manufactured by Evonik Degussa Japan Co., Ltd.)

The pKa of the acid used is as follows.
Sulfuric acid (-3) (reference)
Nitric acid (-1.6)
Phosphoric acid (2.1) (reference)
Formic acid (3.8)
Acetic acid (4.8) (reference)

### Example 2. HPLC measurement

As devices, HPLC (Waters 600, Nihon Waters K.K.), a detector (Waters 490E, Nihon Waters K.K.), a printer (Chromatopak C-R6A, Shimadzu Corporation), and a column (SUPELCOSIL LC18, SUPELCO, Inc.) were used. Measurement was performed under the following conditions: solvent acetonitrile:water:acetic acid=80:20:1 (volume ratio); flow rate 2 ml/ml; temperature 25°C; absorbance 280 nm.

### pH Measurement

The pH of silica was measured according to the following procedure.

A 5 wt% silica-water suspension was prepared using ion-exchanged water, and the electrical potential was measured using glass electrodes to determine the pH.

### Example 3. Inhibition of decarboxylation reaction in cases of addition of 1 wt% or 10 wt% various acids

10 g of a non-heated cashew nut shell liquid were taken in each beaker, and a 1 wt% sulfuric acid, a 1 wt% nitric acid, 1 wt% and 10 wt% phosphoric acids, 1 wt% and 10 wt% formic acids, and 1 wt% and 10 wt% acetic acids were added thereto, respectively, followed by stirring to homogenize each mixture.

The respective beakers were sealed and then left to stand in a thermostat chamber at 80°C, and 4 days later, samples were collected and subjected to composition analyses by HPLC.

An anacardic acid (AA) is converted into a cardanol (CD) by a decarboxylation reaction, and hence Tables 1 and 2 describe the compositions of anacardic acid, cardanol, and cardol before and after the beakers were left to stand in the thermostat chamber. Depending on the number of unsaturated bonds in a fat chain which binds to an aromatic ring and has 15 carbon atoms, there are mainly the following three types of each of anacardic acid, cardanol, and cardol (anacardic acid; AA15:1, AA15:2, and AA15:3, cardanol; CD15:1, CD15:2, and CD15:3, cardol;15:1, 15:2, and 15:3).

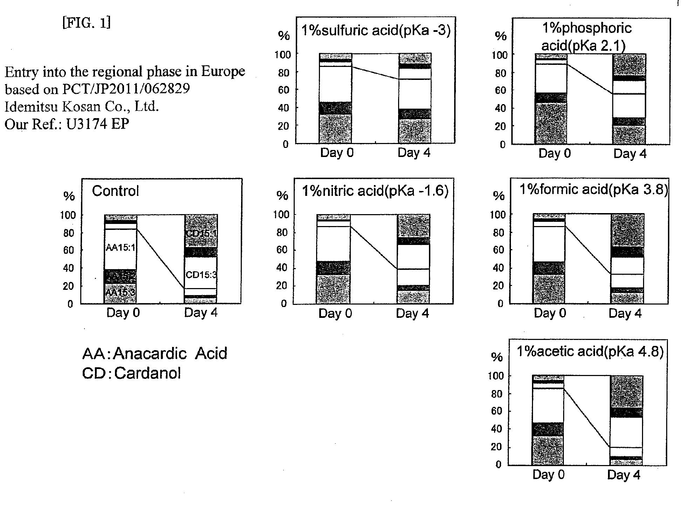

In the cases where each acid was added in an amount of 1 wt% (FIG. 1 and Table 1), the ratios of the decarboxylation (anacardic acid→cardanol) were found to decrease 4 days later depending on the levels of the pKa. In the case of an acetic acid having a pKa of 4.8, the decarboxylation ratio after the addition of the 1 wt% acetic acid was almost the same as that in the case of the addition of no acid (control).

Note that cardol is listed on Table 1, but not in FIG.1.

**[Table 1-1]**

| | | Day 0 | Day 0 (%) | Day 4 | Day 4 (%) |
|---|---|---|---|---|---|
| Control | Anacardic acid C15:3 | 1878 | 16.9 | 753 | 5.4 |
| | Anacardic acid C15:2 | 1335 | 12.0 | 311 | 2.2 |
| | Anacardic acid C15:1 | 3849 | 34.5 | 955 | 6.8 |
| | cardanol C15:3 | 533 | 4.8 | 4213 | 30.0 |
| | cardanol C15:2 | 213 | 1.9 | 1320 | 9.4 |
| | cardanol C15:1 | 591 | 5.3 | 4281 | 30.5 |
| | cardol C15:3 | 1784 | 16.0 | 1700 | 12.1 |
| | cardol C15:2 | 542 | 4.9 | 524 | 3.7 |
| | cardol C15:1 | 422 | 3.8 | 0 | 0.0 |
| | Total | 11147 | 100.0 | 14058 | 100.0 |
| | | | | | |

| | | Day 0 | Day 0 (%) | Day 4 | Day 4 (%) |
|---|---|---|---|---|---|
| 1%sulfuric acid | Anacardic acid C15:3 | 2865 | 25.7 | 2600 | 22.4 |
| | Anacardic acid C15:2 | 1140 | 10.2 | 1099 | 9.5 |
| | Anacardic acid C15:1 | 3522 | 31.7 | 3176 | 27.4 |
| | cardanol C15:3 | 453 | 4.1 | 1119 | 9.6 |
| | cardanol C15:2 | 200 | 1.8 | 396 | 3.4 |
| | cardanol C15:1 | 530 | 4.8 | 1214 | 10.5 |
| | cardol C15:3 | 1601 | 14.4 | 1507 | 13.0 |
| | cardol C15:2 | 451 | 4.1 | 483 | 4.2 |
| | cardol C15:1 | 365 | 3.3 | 0 | 0.0 |
| | Total | 11126 | 100.0 | 11594 | 100.0 |
| | | | | | |

| | | Day 0 | Day 0 (%) | Day 4 | Day 4 (%) |
|---|---|---|---|---|---|
| 1%nitric acid | Anacardic acid C15:3 | 3485 | 25.8 | 1743 | 12.5 |
| | Anacardic acid C15:2 | 1464 | 10.9 | 753 | 5.4 |
| | Anacardic acid C15:1 | 4176 | 31.0 | 2142 | 15.3 |
| | cardanol C15:3 | 578 | 4.3 | 3149 | 22.5 |
| | cardanol C15:2 | 239 | 1.8 | 976 | 7.0 |
| | cardanol C15:1 | 628 | 4.7 | 3097 | 22.2 |
| | cardol C15:3 | 1873 | 13.9 | 1607 | 11.5 |
| | cardol C15:2 | 581 | 4.3 | 509 | 3.6 |
| | cardol C15:1 | 458 | 3.4 | 0 | 0.0 |
| | Total | 13483 | 100.0 | 13976 | 100.0 |

**[Table 1-2]**

| | | Day 0 | Day 0(%) | Day 4 | Day 4 (%) |
|---|---|---|---|---|---|
| 1% phosphoric acid | Anacardic acid C15:3 | 5077 | 37.1 | 2530 | 17.5 |
| | Anacardic acid C15:2 | 1189 | 8.7 | 1075 | 7.5 |
| | Anacardic acid C15:1 | 3633 | 26.6 | 3143 | 21.8 |
| | cardanol C15:3 | 485 | 3.5 | 1871 | 13.0 |
| | cardanol C15:2 | 199 | 1.5 | 565 | 3.9 |
| | cardanol C15:1 | 550 | 4.0 | 2994 | 20.8 |
| | cardol C15:3 | 1699 | 12.4 | 1722 | 11.9 |
| | cardol C15:2 | 477 | 3.5 | 521 | 3.6 |
| | cardol C15:1 | 373 | 2.7 | 0 | 0.0 |
| | Total | 13680 | 100.0 | 14421 | 100.0 |
| | | | | | |

| | | Day 0 | Day 0 (%) | Day 4 | Day 4 (%) |
|---|---|---|---|---|---|
| 1%formic acid | Anacardic acid C15:3 | 3003 | 25.7 | 1157 | 10.1 |
| | Anacardic acid C15:2 | 1207 | 10.3 | 505 | 4.4 |
| | Anacardic acid C15:1 | 3637 | 31.1 | 1453 | 12.6 |
| | cardanol C15:3 | 503 | 4.3 | 1706 | 14.8 |
| | cardanol C15:2 | 207 | 1.8 | 1096 | 9.5 |
| | cardanol C15:1 | 550 | 4.7 | 3495 | 30.4 |
| | cardol C15:3 | 1687 | 14.4 | 1607 | 14.0 |
| | cardol C15:2 | 512 | 4.4 | 494 | 4.3 |
| | cardol C15:1 | 395 | 3.4 | 0 | 0.0 |
| | Total | 11701 | 100.0 | 11515 | 100.0 |
| | | | | | |

| | | Day 0 | Day 0 (%) | Day 4 | Day 4 (%) |
|---|---|---|---|---|---|
| 1% acetic acid | Anacardic acid C15:3 | 3006 | 25.5 | 840 | 6.1 |
| | Anacardic acid C15:2 | 1263 | 10.7 | 352 | 2.6 |
| | Anacardic acid C15:1 | 3641 | 30.9 | 1133 | 8.2 |
| | cardanol C15:3 | 518 | 4.4 | 3945 | 28.7 |
| | cardanol C15:2 | 208 | 1.8 | 1250 | 9.1 |
| | cardanol C15:1 | 558 | 4.7 | 4065 | 29.6 |
| | cardol C15:3 | 1687 | 14.3 | 1670 | 12.1 |
| | cardol C15:2 | 515 | 4.4 | 493 | 3.6 |
| | cardol C15:1 | 396 | 3.4 | 0 | 0.0 |
| | Total | 11792 | 100.0 | 13747 | 100.0 |

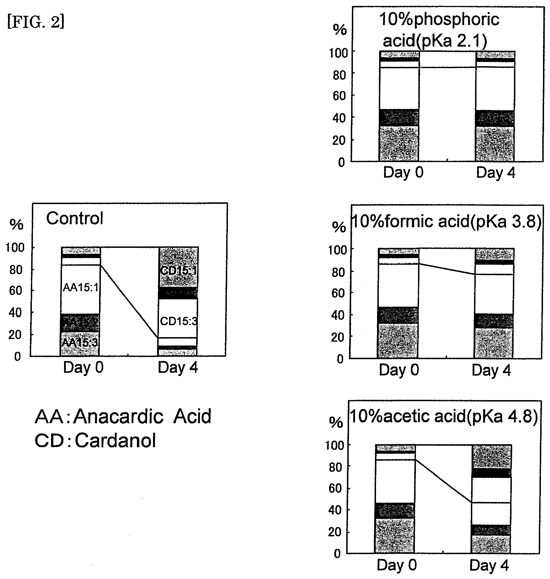

On the other hand, further experiments were performed using the acids each having a relatively low pKa value in an increased addition amount of 10 wt% (FIG. 2 and Table 2), and as a result, the contents of the cardanol components were found to significantly decrease 4 days later up to a formic acid having a pKa of 3.8, and the increases in the addition amounts were found to provide significant effects.

Note that cardol is listed on Table 2, but not in FIG.2.

**[Table 2]**

| | | Day 0 | Day 0 (%) | Day 4 | Day 4 (%) |
|---|---|---|---|---|---|
| Control | Anacardic acid C15:3 | 1878 | 16.9 | 753 | 5.4 |
| | Anacardic acid C15:2 | 1335 | 12.0 | 311 | 2.2 |
| | Anacardic acid C15:1 | 3849 | 34.5 | 955 | 6.8 |
| | cardanol C15:3 | 533 | 4.8 | 4213 | 30.0 |
| | cardanol C15:2 | 213 | 1.9 | 1320 | 9.4 |
| | cardanol C15:1 | 591 | 5.3 | 4281 | 30.5 |
| | cardol C15:3 | 1784 | 16.0 | 1700 | 12.1 |
| | cardol C15:2 | 542 | 4.9 | 524 | 3.7 |
| | cardol C15:1 | 422 | 3.8 | 0 | 0.0 |
| | Total | 11147 | 100.0 | 14058 | 100.0 |
| | | | | | |

| | | Day 0 | Day 0 (%) | Day 4 | Day 4 (%) |
|---|---|---|---|---|---|
| 10% phosphoric acid | Anacardic acid C15:3 | 2891 | 25.7 | 3277 | 26.0 |
| | Anacardic acid C15:2 | 1218 | 10.8 | 1440 | 11.4 |
| | Anacardic acid C15:1 | 3467 | 30.8 | 3924 | 31.2 |
| | Cardanol C15:3 | 474 | 4.2 | 593 | 4.7 |
| | cardanol C15:2 | 185 | 1.6 | 249 | 2.0 |
| | cardanol C15:1 | 516 | 4.6 | 601 | 4.8 |
| | cardol C15:3 | 1614 | 14.4 | 1612 | 12.8 |
| | cardol C15:2 | 489 | 4.4 | 504 | 4.0 |
| | cardol C15:1 | 386 | 3.4 | 381 | 3.0 |
| | Total | 11240 | 100.0 | 12580 | 100.0 |
| | | | | | |

| | | Day 0 | Day 0 (%) | Day 4 | Day 4 (%) |
|---|---|---|---|---|---|
| 10% formic acid | Anacardic acid C15:3 | 3004 | 25.8 | 2376 | 23.5 |
| | Anacardic acid C15:2 | 1216 | 10.4 | 990 | 9.8 |
| | Anacardic acid C15:1 | 3626 | 31.1 | 2869 | 28.4 |
| | cardanol C15:3 | 503 | 4.3 | 832 | 8.2 |
| | cardanol C15:2 | 207 | 1.8 | 291 | 2.9 |
| | cardanol C15:1 | 541 | 4.6 | 863 | 8.5 |
| | cardol C15:3 | 1679 | 14.4 | 1237 | 12.3 |
| | cardol C15:2 | 475 | 4.1 | 382 | 3.8 |
| | cardol C15:1 | 396 | 3.4 | 257 | 2.5 |
| | Total | 11646 | 100.0 | 10,098 | 100.0 |
| | | | | | |

| | | Day 0 | Day 4 (%) | Day 4 | Day 4 (%) |
|---|---|---|---|---|---|
| 10% acetic acid | Anacardic acid C15:3 | 2786 | 25.6 | 1872 | 15.0 |
| | Anacardic acid C15:2 | 1114 | 10.2 | 804 | 6.4 |
| | Anacardic acid C15:1 | 3384 | 31.1 | 2307 | 18.5 |
| | cardanol C15:3 | 462 | 4.2 | 2435 | 19.5 |
| | cardanol C15:2 | 177 | 1.6 | 741 | 5.9 |
| | cardanol C15:1 | 519 | 4.8 | 2295 | 18.4 |
| | cardol C15:3 | 1567 | 14.4 | 1555 | 12.4 |
| | cardol C15:2 | 495 | 4.6 | 479 | 3.8 |
| | cardol C15:1 | 375 | 3.4 | 0 | 0.0 |
| | Total | 10878 | 100.0 | 12488 | 100.0 |

### Example 4 (reference). Inhibition of decarboxylation reaction in cases of addition of various concentrations of sulfuric acids (pKa=-3)

10 g of a non-heated cashew nut shell liquid was taken in each beaker, and 0.1 wt%, 0.6 wt%, and 1 wt% sulfuric acids were added thereto, respectively, followed by stirring to homogenize each mixture.

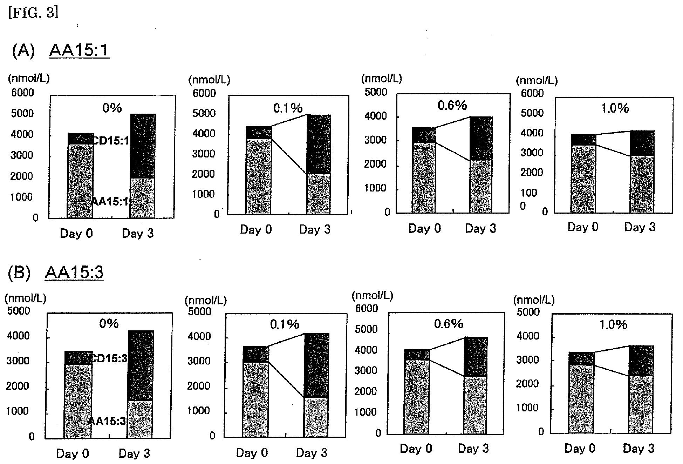

The respective beakers were sealed and then left to stand in a thermostat chamber at 80°C, and 3 days later, samples were collected and subjected to composition analyses by HPLC. The analysis results are separately shown for the anacardic acid 15:1 and the anacardic acid 15:3 as the concentrations in the analyses (nmol/L) (Table 3, left and right columns and FIG. 3A and 3B). As is clear from the figure, the decarboxylation reaction decreased when a sulfuric acid was added in an amount of 0.6 wt%, and the decarboxylation reaction significantly decreased when a sulfuric acid was added in an amount of 1 wt%.

**[Table 3]**

| Control | | | | | Control | | | |
|---|---|---|---|---|---|---|---|---|
| | | Day0 | Day3 | | | | Day0 | Day3 |
| C15:3 | AA | 2938 | 1543 | | C15:1 | AA | 3604 | 1978 |
| | CD | 588 | 2722 | | | CD | 564 | 3064 |
| | | | | | | | | |

| 0.1%sulfuric acid | | | | | 0.1%sulfuric acid | | | |
|---|---|---|---|---|---|---|---|---|
| | | Day0 | Day3 | | | | Day0 | Day3 |
| C15:3 | AA | 3006 | 1614 | | C15:1 | AA | 3788 | 2074 |
| | CD | 612 | 2571 | | | CD | 603 | 2896 |
| | | | | | | | | |

| 0.6%sulfuric acid | | | | | 0.6%sulfuric acid | | | |
|---|---|---|---|---|---|---|---|---|
| | | Day0 | Day3 | | | | Day0 | Day3 |
| C15:3 | AA | 2973 | 1745 | | C15:1 | AA | 3648 | 2264 |
| | CD | 606 | 2244 | | | CD | 568 | 2562 |
| | | | | | | | | |

| 1.0%sulfuric acid | | | | | 1.0%sulfuric acid | | | |
|---|---|---|---|---|---|---|---|---|
| | | Day0 | Day3 | | | | Day0 | Day3 |
| C15:3 | AA | 2858 | 2426 | | C15:1 | AA | 3505 | 2988 |
| | CD | 570 | 1213 | | | CD | 546 | 1287 |

### Example 5 (reference). Inhibition of decarboxylation reaction in non-heated cashew nut shell liquid preparation (preliminary addition of phosphoric acid to non-heated cashew nut shell liquid)

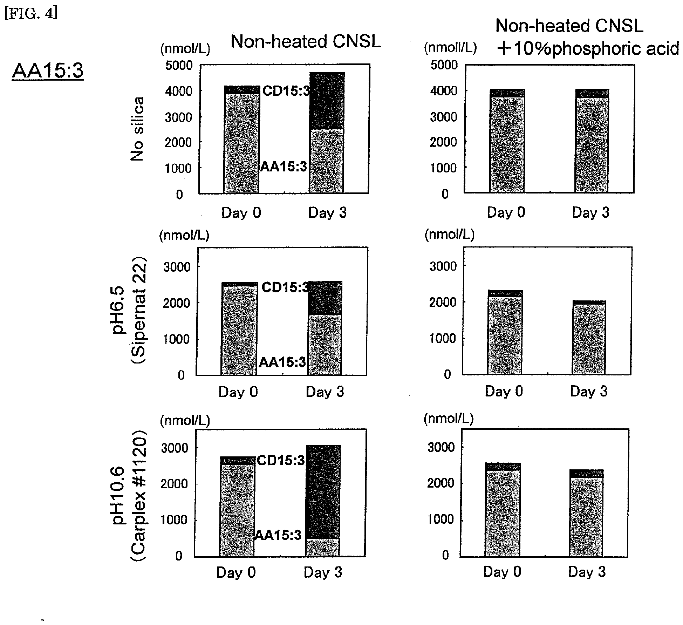

10 g of silica (Sipernat 22, Carplex #1120) were taken in each beaker, and 20 g of a non-heated cashew nut shell liquid or 20 g of a non-heated cashew nut shell liquid containing a 10 wt% phosphoric acid were added thereto, followed by stirring and mixing to homogenize each mixture. The respective beakers were sealed and then left to stand in a thermostat chamber at 80°C, and 3 days later, samples were collected and subjected to composition analyses by HPLC. The HPLC analyses were performed by: adjusting the concentrations of the collected samples with ethyl acetate so as to be 5 mg (including silica) /ml; then centrifuging the samples under conditions of 15,000 rpm and 5 min; and performing measurement for the resultant supernatants. The analysis results are shown as the concentrations (nmol/L) of the anacardic acid 15:3 in the analyses (Table 4 and FIG. 4).

In the case where silica, i.e., Sipernat 22 having an about neutral pH, was used as the carrier, the decarboxylation ratio was almost the same as or lower than that in the case of using no carrier, while in the case where an alkaline Carplex #1120 was used as the carrier, the decarboxylation ratio was found to apparently increase.

On the other hand, in the case where non-heated cashew nut shell liquid containing a 10 wt% phosphoric acid was used, the decarboxylation reaction was found to be inhibited regardless of the use of the carrier and of the pH of silica used as the carrier.

The results are shown Table 4 below.

**[Table 4]**

| Control | | | | | 10% phosphoric acid | | | |
|---|---|---|---|---|---|---|---|---|
| | | Day 0 | Day 3 | | | | Day 0 | Day 3 |
| C15:3 | Anacardic acid | 3852 | 2518 | | C15:3 | Anacardic acid | 3757 | 3724 |
| | Cardanol | 315 | 2170 | | | Cardanol | 279 | 310 |
| | | | | | | | | |

| S22/Control | | | | | S22/10% phosphoric acid | | | |
|---|---|---|---|---|---|---|---|---|
| | | Day 0 | Day 3 | | | | Day 0 | Day 3 |
| C15:3 | Anacardic acid | 2444 | 1664 | | C15:3 | Anacardic acid | 2136 | 1936 |
| | Cardanol | 84 | 910 | | | Cardanol | 170 | 90 |
| | | | | | | | | |

| 1120/Control | | | | | 1120/10% phosphoric acid | | | |
|---|---|---|---|---|---|---|---|---|
| | | Day 0 | Day 3 | | | | Day 0 | Day 3 |
| C15:3 | Anacardic acid | 2548 | 512 | | C15:3 | Anacardic acid | 2375 | 2168 |
| | Cardanol | 210 | 2530 | | | Cardanol | 190 | 205 |

### Example 6 (reference). Inhibition of decarboxylation reaction in non-heated cashew nut shell liquid preparation (addition of phosphoric acid to carrier in advance)

150 g of a Sipernat 22 were collected in a plastic bag, and 9 ml of an 85% phosphoric acid was charged in a vaporizer and sprayed thereto while stirring. The pH of the Sipernat 22 supplemented with the phosphoric acid thus prepared was found to be 2.0 by measurement.

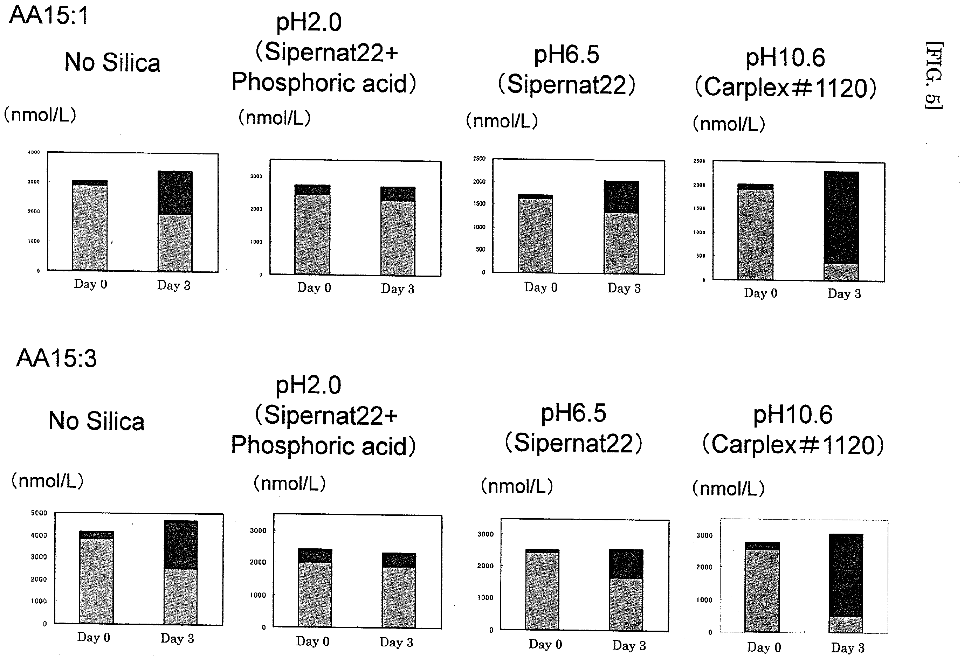

10 g of silica (Sipernat 22 supplemented with phosphoric acid, Sipernat 22, and Carplex #1120) were taken in each beaker, and 20 g of a non-heated cashew nut shell liquid were added thereto, respectively, followed by stirring and mixing to homogenize each mixture. The respective beakers were sealed and then left to stand in a thermostat chamber at 80°C, and 3 days later, samples were collected and subjected to composition analyses by HPLC. The HPLC analyses were performed by: adjusting the concentrations of the collected samples with ethyl acetate so as to be 5 mg (including silica) /ml; then centrifuging the samples under conditions of 15,000 rpm and 5 min; and performing measurement for the resultant supernatants. The analysis results are shown as the concentrations (nmol/L) of the anacardic acid 15:1 and the anacardic acid 15:3 in the analyses (Table 5 and FIG. 5).

The results are shown Table 5 below.

**[Table 5]**

| AA15:1 | | | | | | | | |
|---|---|---|---|---|---|---|---|---|
| No silica | | | | | pH2.0(S22/phosphoric acid) | | | |
| | | Day 0 | Day 3 | | | | Day 0 | Day 3 |
| C15:1 | Anacardic acid | 2803 | 1919 | | C15:1 | Anacardic acid | 2450 | 2271 |
| | Cardanol | 148 | 1473 | | | Cardanol | 295 | 470 |

| pH0.5/S22 | | | | | pH10/1120 | | | |
|---|---|---|---|---|---|---|---|---|
| | | Day 0 | Day 3 | | | | Day 0 | Day 3 |
| C15:1 | Anacardic acid | 1641 | 1354 | | C15:1 | Anacardic acid | 1902 | 387 |
| | Cardanol | 82 | 668 | | | Cardanol | 102 | 1912 |

| AA15:3 | | | | | | | | |
|---|---|---|---|---|---|---|---|---|
| No silica | | | | | pH2.0(S22/phosphoric acid) | | | |
| | | Day 0 | Day 3 | | | | Day 0 | Day 3 |
| C15:3 | Anacardic acid | 3652 | 2518 | | C15:3 | Anacardic acid | 1996 | 1850 |
| | Cardanol | 315 | 2170 | | | Cardanol | 409 | 440 |

| pH6.5./S22 | | | | | pH10.6/1120 | | | |
|---|---|---|---|---|---|---|---|---|
| | | Day 0 | Day 3 | | | | Day 0 | Day 3 |
| C15:3 | Anarcadic acid | 2444 | 1664 | | C15:3 | Anacardic acid | 2548 | 512 |
| | Cardanol | 84 | 910 | | | Cardanol | 210 | 2530 |

The results reveal that the decarboxylation reaction is more inhibited as the pH of the carrier (silica) is lower.

In view of the foregoing, it is found that the acids to be used for the decarboxylation reaction are not necessary to be preliminarily added to the non-heated cashew nut shell liquids, and the acids may be added to the carriers in advance.

### Example 7 (reference). Inhibition of decarboxylation reaction in feed

To 100 g of a feed (standard feed for breeding young cattle: SDC No.2 Nippon Formula Feed Mfg Co., Ltd.) were added 1 wt% two kinds of the silica preparations produced in Example 5 (10g of Sipernat 22 + 20 g of non-heated cashew nut shell liquid or 20 g of non-heated cashew nut shell liquid containing 10 wt% phosphoric acid), to thereby produce feeds each containing the non-heated cashew nut shell liquid.

The respective resultants were sealed and then left to stand in a thermostat chamber at 80°C, and 3 days later, samples were collected and subjected to composition analyses by HPLC.

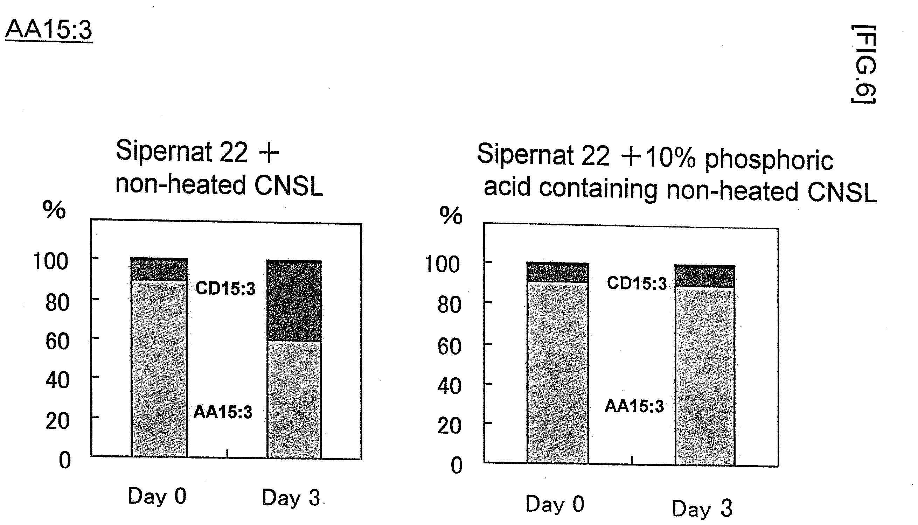

The HPLC analyses were performed by: extracting soluble parts with ethyl acetate from the feeds used in the test; filtrating and drying the extracts; adjusting the concentrations so as to be 5 mg/ml; and performing measurement. The analysis results show the compositions of the anacardic acid and the cardanol before and after the samples were left to stand in the thermostat chamber as for the anacardic acid 15:3 (Table 6 and FIG. 6).

The results are shown in Table 6 below.

**[Table 6]**

| | | Day 0 | | | | Day 3 | | | |
|---|---|---|---|---|---|---|---|---|---|
| | | Anacardic acid 15:3 | | Cardanol 15:3 | | Anacardic acid 15:3 | | Cardanol 15:3 | |
| Sipernat22 + Non-heated CNSL | area | 16232 | | 1752 | | 9456 | | 5903 | |
| | ppm | 386 | | 37 | | 225 | | 123 | |
| | n mol | 1129 | 90% | 123 | 10% | 658 | 61% | 414 | 39% |
| Sipernat22 + 10% phosphoric acid containing Non-heated CNSL | area | 14356 | | 1489 | | 13389 | | 1411 | |
| | ppm | 341 | | 31 | | 318 | | 29 | |
| | n mol | 998 | 91% | 104 | 9% | 931 | 90% | 99 | 10% |

This suggests that, in the case of using the non-heated cashew nut shell liquid containing a 10 wt% phosphoric acid, the anacardic acid is present stably without the progress of the decarboxylation in the feed.

### Example 8 (reference). Production of feed supplemented with non-heated cashew nut shell liquid (use of organic carrier)

A standard feed was used as a carrier to produce feeds for chicken and cattle. The formulations are not limited to the following examples.

### (1) Production of feed for broiler

| | |
|---|---|
| Standard feed for broiler in early fattening phase: SDB No.1 Nippon Formula Feed Mfg Co., Ltd. | 99.5 wt% |
| Non-heated cashew nut shell liquid (10 wt% phosphoric acid) | 0.05 wt% |

When the components are mixed, a powder is obtained, and the solidification at about 20°C can be prevented.

### (2) Production of feed for cattle

| | |
|---|---|
| Standard feed for breeding young cattle: SDC No.2 Nippon Formula Feed Mfg Co., Ltd. | 99.5 wt% |
| Non-heated cashew nut shell liquid (10 wt% phosphoric acid) | 0.05wt% |

When the components are mixed, a powder is obtained, and the solidification at about 20°C can be prevented.

### Industrial Applicability

The addition of an acid to a non-heated cashew nut shell liquid can inhibit foam formation due to decarboxylation. Further, the blending of an inorganic carrier in the non-heated cashew nut shell liquid supplemented with the acid can prevent the solidification at about 20°C. Therefore, the non-heated cashew nut shell liquid can be easily used for industrial applications.

## Claims

1. A method of producing a non-heated cashew nut shell liquid preparation, comprising mixing one or more acid components with one or more carriers and further mixing the carrier mixed with the acid component with the non-heated cashew nut shell liquid, wherein the non-heated cashew nut shell liquid preparation comprises:
one or more carriers, and
a composition comprising one or more acids and a non-heated cashew nut shell liquid, wherein decarboxylation of an anacardic acid in the non-heated cashew nut is inhibited by the one or more acids, wherein the one or more acids is/are selected from formic acid, malic acid, citric acid, succinic acid, tartaric acid, nitric acid, malonic acid, glutaric acid, adipic acid, fumaric acid, maleic acid, phthalic acid, isophthalic acid, phytic acid, terephthalic acid, lactic acid, sulfamic acid, hydroxyacetic acid, phosphonic acid, and gluconic acid, and wherein the amount of the acid is 0.5 wt% or more with respect to the non-heated cashew nut shell liquid.

2. The method of producing a non-heated cashew nut shell liquid preparation according to claim 1, wherein the carrier is an inorganic carrier and has a specific surface area of 500 m³/g or less.

3. The method of producing a non-heated cashew nut shell liquid preparation according to claim 1 or 2, wherein the carrier is selected from the group consisting of a silicic acid and a salt thereof, vermiculite, diatomaceous earth, talc, kaolin, and bentonite.

4. The method of producing a non-heated cashew nut shell liquid preparation according to claim 2, wherein the carrier is silica.

5. The method of producing a non-heated cashew nut shell liquid preparation according to claim 4, wherein the blending ratio (weight ratio) is silica/non-heated cashew nut shell liquid=1/3.0 to 1/0.1.

6. A method of inhibiting a decarboxylation reaction of an anacardic acid in a non-heated cashew nut shell liquid, comprising adding one or more acids to the non-heated cashew nut shell liquid, wherein the one or more acids is/are selected from formic acid, malic acid, citric acid, succinic acid, tartaric acid, nitric acid, malonic acid, glutaric acid, adipic acid, fumaric acid, maleic acid, phthalic acid, isophthalic acid, phytic acid, terephthalic acid, lactic acid, sulfamic acid, hydroxyacetic acid, phosphonic acid, and gluconic acid, and wherein the amount of the acid is 0.5 wt% or more with respect to the non-heated cashew nut shell liquid.

7. Use of one or more acids for inhibiting a decarboxylation reaction of an anacardic acid in a non-heated cashew nut shell liquid by adding the one or more acids to the non-heated cashew nut shell liquid, wherein the one or more acids is/are selected from formic acid, malic acid, citric acid, succinic acid, tartaric acid, nitric acid, malonic acid, glutaric acid, adipic acid, fumaric acid, maleic acid, phthalic acid, isophthalic acid, phytic acid, terephthalic acid, lactic acid, sulfamic acid, hydroxyacetic acid, phosphonic acid, and gluconic acid, and wherein the amount of the acid is 0.5 wt% or more with respect to the non-heated cashew nut shell liquid.

8. The method according to claim 1 or 6 or the use according to claim 7, wherein the one or more acids is/are selected from formic acid, malic acid, citric acid, succinic acid, tartaric acid, malonic acid, glutaric acid, adipic acid, fumaric acid, maleic acid, phthalic acid, isophthalic acid, phytic acid, terephthalic acid, lactic acid, hydroxyacetic acid, gluconic acid and nitric acid.

9. The method according to claim 1 or 6 or the use according to claim 7, wherein the one or more acids is/are selected from malic acid, citric acid, tartaric acid and nitric acid.

10. The method according to claim 1 or 6 or the use according to claim 7, wherein the amount of the acid is 5 to 10 wt% for the acids having a pKa of higher than 2.

## Patentansprüche

1. Ein Verfahren zur Herstellung einer Zubereitung aus nicht erwärmter Cashewnussschalenflüssigkeit, umfassend Mischen einer oder mehrerer Säurekomponenten mit einem oder mehreren Trägern und weiter Mischen des mit der Säurekomponente gemischten Trägers mit der nicht erwärmten Cashewnussschalenfl üssigkeit,
wobei die Zubereitung aus nicht erwärmter Cashewnussschalenflüssigkeit umfasst:
einen oder mehrere Träger, und
eine Zusammensetzung, umfassend eine oder mehrere Säuren und eine nicht erwärmte Cashewnussschalenflüssigkeit, wobei die Decarboxylierung einer Anacardinsäure in der nicht erwärmten Cashewnuss durch die eine oder mehreren Säuren gehemmt wird, wobei die eine oder mehreren Säuren ausgewählt ist/sind aus Ameisensäure, Äpfelsäure, Zitronensäure, Bernsteinsäure, Weinsäure, Salpetersäure, Malonsäure, Glutarsäure, Adipinsäure, Fumarsäure, Maleinsäure, Phthalsäure, Isophthalsäure, Phytinsäure, Terephthalsäure, Milchsäure, Sulfaminsäure, Hydroxyessigsäure, Phosphonsäure und Gluconsäure, und wobei die Menge der Säure 0,5 Gew.-% oder mehr, bezogen auf die nicht erwärmte Cashewnussschalenflüssigkeit, beträgt.

2. Das Verfahren zur Herstellung einer Zubereitung aus nicht erwärmter Cashewnussschalenflüssigkeit gemäß Anspruch 1, wobei der Träger ein anorganischer Träger ist und eine spezifische Oberfläche von 500 m³/g oder weniger aufweist.

3. Das Verfahren zur Herstellung einer Zubereitung aus nicht erwärmter Cashewnussschalenflüssigkeit gemäß Anspruch 1 oder 2, wobei der Träger ausgewählt ist aus der Gruppe bestehend aus einer Kieselsäure und einem Salz davon, Vermiculit, Diatomeenerde, Talkum, Kaolin und Bentonit.

4. Das Verfahren zur Herstellung einer Zubereitung aus nicht erwärmter Cashewnussschalenflüssigkeit gemäß Anspruch 2, wobei der Träger Siliciumdioxid ist.

5. Das Verfahren zur Herstellung einer Zubereitung aus nicht erwärmter Cashewnussschalenflüssigkeit gemäß Anspruch 4, wobei das Mischverhältnis (Gewichtsverhältnis) Siliziumdioxid/ nicht erwärmte Cashewnussschalenflüssigkeit = 1/3,0 bis 1/0,1 beträgt.

6. Ein Verfahren zum Hemmen einer Decarboxylierungsreaktion einer Anacardinsäure in einer nicht erwärmten Cashewnussschalenflüssigkeit, umfassend das Hinzufügen einer oder mehrerer Säuren zu der nicht erwärmten Cashewnussschalenflüssigkeit, wobei die eine oder mehreren Säuren ausgewählt ist/sind aus Ameisensäure, Äpfelsäure, Zitronensäure, Bernsteinsäure, Weinsäure, Salpetersäure, Malonsäure, Glutarsäure, Adipinsäure, Fumarsäure, Maleinsäure, Phthalsäure, Isophthalsäure, Phytinsäure, Terephthalsäure, Milchsäure, Sulfaminsäure, Hydroxyessigsäure, Phosphonsäure und Gluconsäure, und wobei die Menge der Säure 0,5 Gew.-% oder mehr, bezogen auf die nicht erwärmte Cashewnussschalenflüssigkeit, beträgt.

7. Verwendung einer oder mehrerer Säuren zum Hemmen einer Decarboxylierungsreaktion einer Anacardinsäure in einer nicht erwärmten Cashewnussschalenflüssigkeit durch Zugabe der einen oder mehreren Säuren zu der nicht erwärmten Cashewnussschalenflüssigkeit wobei die eine oder mehreren Säuren ausgewählt ist/sind aus Ameisensäure, Äpfelsäure, Zitronensäure, Bernsteinsäure, Weinsäure, Salpetersäure, Malonsäure, Glutarsäure, Adipinsäure, Fumarsäure, Maleinsäure, Phthalsäure, Isophthalsäure, Phytinsäure, Terephthalsäure, Milchsäure, Sulfaminsäure, Hydroxyessigsäure, Phosphonsäure und Gluconsäure, und wobei die Menge der Säure 0,5 Gew.-% oder mehr, bezogen auf die nicht erwärmte Cashewnussschalenflüssigkeit, beträgt.

8. Das Verfahren gemäß Anspruch 1 oder 6 oder die Verwendung gemäß Anspruch 7, wobei die eine oder mehreren Säuren ausgewählt ist/sind aus Ameisensäure, Äpfelsäure, Zitronensäure, Bernsteinsäure, Weinsäure, Malonsäure, Glutarsäure, Adipinsäure, Fumarsäure, Maleinsäure, Phthalsäure, Isophthalsäure, Phytinsäure, Terephthalsäure, Milchsäure, Hydroxyessigsäure, Gluconsäure und Salpetersäure.

9. Das Verfahren gemäß Anspruch 1 oder 6 oder die Verwendung gemäß Anspruch 7, wobei die eine oder mehreren Säuren ausgewählt ist/sind aus Äpfelsäure, Zitronensäure, Weinsäure und Salpetersäure.

10. Das Verfahren gemäß Anspruch 1 oder 6 oder die Verwendung gemäß Anspruch 7, wobei die Menge an Säure 5 bis 10 Gew.-%, für die Säuren mit einem pKa von größer als 2, beträgt.

## Revendications

1. Méthode de production d'une préparation de liquide de coque de noix de cajou non chauffée, comprenant le mélange d'un ou plusieurs composants acides avec un ou plusieurs supports et en outre le mélange du support mélangé avec le composé acide avec le liquide de coque de cajou non chauffé, dans laquelle la préparation de liquide de coque de noix de cajou non chauffé comprend :
un ou plusieurs supports, et
une composition comprenant un ou plusieurs acides et un liquide de coque de cajou non chauffé,
dans laquelle la décarboxylation d'un acide anacardique dans la noix de cajou non chauffée est inhibée par le ou les acides, dans laquelle le ou les acides sont choisis parmi l'acide formique, l'acide malique, l'acide citrique, l'acide succinique, l'acide tartrique, l'acide nitrique, l'acide malonique, l'acide glutarique, l'acide adipique, l'acide fumarique, l'acide maléique, l'acide phtalique, l'acide isophtalique, l'acide phytique, l'acide téréphtalique, l'acide lactique, l'acide sulfamique, l'acide hydroxyacétique, l'acide phosphonique, et l'acide gluconique, et dans laquelle la quantité de l'acide est de 0,5 % en poids ou plus par rapport au liquide de coque de noix de cajou non chauffé.

2. Méthode de production d'une préparation de liquide de coque de noix de cajou non chauffée selon la revendication 1, dans laquelle le support est un support inorganique et a une surface spécifique de 500 m³/g ou moins.

3. Méthode de production d'une préparation de liquide de coque de noix de cajou non chauffée selon la revendication 1 ou 2, dans laquelle le support est choisi dans le groupe constitué par un acide silicique et un sel de celui-ci, la vermiculite, la terre de diatomées, le talc, le kaolin, et la bentonite.

4. Méthode de production d'une préparation de liquide de coque de noix de cajou non chauffée selon la revendication 2, dans lequel le support est la silice.

5. Méthode de production d'une préparation de liquide de coque de noix de cajou non chauffée selon la revendication 4, dans laquelle le rapport de mélange (rapport en poids) silice/liquide de coque de noix de cajou non chauffé est de 1/3,0 à 1/0,1.

6. Méthode d'inhibition d'une réaction de décarboxylation d'un acide anacardique dans un liquide de coque de noix de cajou non chauffé, comprenant l'addition d'un ou plusieurs acides au liquide de coque de noix de cajou non chauffé, dans laquelle le ou les acides sont choisis parmi l'acide formique, l'acide malique, l'acide citrique, l'acide succinique, l'acide tartrique, l'acide nitrique, l'acide malonique, l'acide glutarique, l'acide adipique, l'acide fumarique, l'acide maléique, l'acide phtalique, l'acide isophtalique, l'acide phytique, l'acide téréphtalique, l'acide lactique, l'acide sulfamique, l'acide hydroxyacétique, l'acide phosphonique, et l'acide gluconique, et dans laquelle la quantité de l'acide est de 0,5 % en poids ou plus par rapport au liquide de coque de noix de cajou non chauffé.

7. Utilisation d'un ou plusieurs acides pour inhiber une réaction de décarboxylation d'un acide anacardique dans un liquide de coque de noix de cajou non chauffé par addition du ou des acides au liquide de coque de noix de cajou non chauffé, dans laquelle le ou les acides est/sont choisis parmi l'acide formique, l'acide malique, l'acide citrique, l'acide succinique, l'acide tartrique, l'acide nitrique, l'acide malonique, l'acide glutarique, l'acide adipique, l'acide fumarique, l'acide maléique, l'acide phtalique, l'acide isophtalique, l'acide phytique, l'acide téréphtalique, l'acide lactique, l'acide sulfamique, l'acide hydroxyacétique, l'acide phosphonique, et l'acide gluconique, et dans laquelle la quantité de l'acide est de 0,5 % en poids ou plus par rapport au liquide de coque de noix de cajou non chauffé.

8. Méthode selon la revendication 1 ou 6 ou utilisation selon la revendication 7, dans laquelle le ou les acides est/sont choisis parmi l'acide formique, l'acide malique, l'acide citrique, l'acide succinique, l'acide tartrique, l'acide malonique, l'acide glutarique, l'acide adipique, l'acide fumarique, l'acide maléique, l'acide phtalique, l'acide isophtalique, l'acide phytique, l'acide téréphtalique, l'acide lactique, l'acide hydroxyacétique, l'acide gluconique et l'acide nitrique.

9. Méthode selon la revendication 1 ou 6 ou utilisation selon la revendication 7, dans laquelle le ou les acides est/sont choisis parmi l'acide malique, l'acide citrique, l'acide tartrique et l'acide nitrique.

10. Méthode selon la revendication 1 ou 6 ou utilisation selon la revendication 7, dans laquelle la quantité de l'acide est de 5 à 10 % en poids pour les acides ayant un pKa supérieur à 2.
